# FASCICULE DE BREVET EUROPEEN

(11) **EP 0 940 136 B1**
(45) Date de publication et mention de la délivrance du brevet: **13.10.2004**
(21) Numéro de dépôt: 98403149.2
(22) Date de dépôt: 14.12.1998
(51) Int. Cl.: A61K 7/09

(54) **Agent réducteur à plusieurs composants et procédé de déformation permanente des cheveux le mettant en oeuvre**
Reduktionsmittel von mehreren Komponenten und Verfahren für eine dauerhafte Haarverformung durch die Verwendung desselben
Reducing agent from a plurality of components and process for permanent hair waving using it

(30) Priorité: 30.12.1997 FR 9716717
(43) Date de publication de la demande: 08.09.1999
(73) Titulaire: L'OREAL, 75008 Paris (FR)
(72) Inventeur: Nguyen, Ly-Lan, 94240 l'Hay les Roses (FR); Sabbagh, Anne, 92500 Rueil Malmaison (FR)
(74) Mandataire: Casalonga, Axel

(56) Documents cités:
- EP-A- 0 362 663
- EP-A- 0 377 836
- WO-A-97/04738
- DE-A- 4 317 663

## Description

La présente invention est relative à un agent réducteur à au moins deux composants comprenant une composition contenant un réducteur en solution aqueuse et une composition contenant un agent épaississant particulier les deux compositions étant mélangées avant emploi et le mélange étant utilisé dans un procédé de traitement des cheveux, en vue d'obtenir une déformation permanente de ces derniers et aux procédés mettant en oeuvre cet agent.

L'une des techniques couramment utilisée dans le domaine cosmétique pour imprimer aux cheveux une forme durable consiste à procéder à la déformation des cheveux en mettant en oeuvre un agent de réduction puis un agent oxydant.

La technique la plus usuelle pour obtenir une déformation permanente des cheveux consiste dans un premier temps à réaliser l'ouverture des liaisons disulfure (S-S) de la kératine (cystine) à l'aide d'une composition contenant un agent réducteur, puis après avoir de préférence rincé la chevelure ainsi traitée, à reconstituer dans un second temps lesdites liaisons disulfure en appliquant sur les cheveux préalablement mis sous tension, par des bigoudis ou autres, ou mis en forme ou lissés par d'autres moyens, une composition oxydante encore appelée "fixateur" de façon à donner, en définitive, la forme recherchée aux cheveux.

Cette technique permet ainsi de réaliser, soit l'ondulation des cheveux, soit leur défrisage ou leur décrêpage ou encore leur lissage.

Cette nouvelle forme, imposée aux cheveux par un traitement chimique, est durable dans le temps pendant quelques semaines et résiste notamment à l'action des lavages à l'eau ou aux shampoings et ceci par opposition aux techniques mettant en oeuvre des produits de coiffage entraînant une déformation temporaire, telle que la mise en plis, une telle déformation disparaissant au coiffage ou au shampoing.

Les compositions réductrices généralement utilisées pour la première étape d'une opération de permanente contiennent à titre d'agents réducteurs des sulfites, des bisulfites ou de préférence des thiols. Parmi ceux-ci, on peut citer plus particulièrement la cystéine et ses dérivés, la cystéamine et ses dérivés, l'acide thiolactique, l'acide thioglycolique ainsi que ses esters, notamment le thioglycolate de glycérol. L'acide thioglycolique est particulièrement efficace et constitue le produit le plus utilisé pour réduire les liaisons disulfures de la kératine.

Pour certaines techniques de permanentes comme par exemple lors de procédés de déformation sans bigoudis, lors d'un défrisage, il est préférable d'utiliser des agents réducteurs suffisamment épaissis afin d'en faciliter l'application, de permettre une meilleure localisation du produit sur la chevelure, d'éviter que la composition réductrice ne coule, et de permettre un maintien des cheveux dans la position désirée.

Néanmoins, la formulation de réducteurs épaissis est particulièrement difficile en raison de fréquents problèmes d'instabilité dans le temps. Plusieurs phénomènes se produisent généralement : on observe notamment souvent une chute de la viscosité du produit, et/ou une diminution du titre en agent réducteur, et/ou une apparition d'une odeur désagréable, soit tous ces désagréments en même temps.

Si l'on sépare le système épaississant du réducteur des fibres kératiniques (système multicompartiments) on se heurte à la difficulté d'obtenir un épaississement homogène conséquent suffisamment rapidement et facilement après mélange.

Les documents WO 97/04738 et DE 43 17 663 divulguent des compositions réductrices pour permanentes, éventuellement sous forme de deux composants conservés séparément et mélangés directement avant emploi, contenant éventuellement, dans un des composants, un agent épaississant polymérique choisi parmi l'amidon, le poly(acide acrylique), les dérivés de cellulose et les alginates. Aucun de ces documents ne divulgue les polymères épaississants particuliers en dispersion, décrits ci-après.

La demande EP 0 362 663 concerne également des compositions réductrices pour permanentes, éventuellement sous forme de deux composants conservés séparément et mélangés directement avant emploi. L'épaississement de ces compositions se fait principalement à l'aide d'agents épaississants permettant l'ajustement du point de liquéfaction de la composition à une température comprise entre 20 et 30 °C, tels que des alcools gras éthoxylés, des esters de polyols et d'acides gras, des alcools gras et/ou des paraffines.

La présente invention permet de résoudre ces différents problèmes en mettant à disposition un agent réducteur à deux composants comprenant un premier composant constitué par une composition contenant un réducteur thiolé en solution aqueuse et un second composant constitué par une composition contenant un polymère épaississant sous une forme particulière.

Les compositions de la présente invention présentent notamment l'avantage d'être très facilement et rapidement réalisables, permettant ainsi d'obtenir une composition réductrice épaissie et homogène pratiquement immédiatement, par simple mélange des deux composants.

De plus, la composition réductrice de la présente invention présente une texture particulièrement adaptée à toutes les applications et notamment à celle sur cheveux non enroulés sur bigoudis; cette composition étant facilement applicable, elle ne coule pas et permet le maintient des cheveux dans la position désirée.

Un objet de l'invention est constitué par un agent réducteur à au moins deux composants comprenant une composition contenant un réducteur en solution aqueuse et une composition contenant un polymère épaississant en dispersion aqueuse ou huileuse ou en émulsion inverse.

Un autre objet de l'invention est constitué par un procédé de déformation permanente des cheveux mettant en oeuvre une composition obtenue par mélange avant emploi des deux composants de l'agent réducteur susmentionné; suivi par l'application d'une composition oxydante.

D'autres objets de l'invention apparaîtront à la lecture de la description et des exemples qui suivent.

L'agent réducteur conforme à l'invention est essentiellement caractérisé par le fait qu'il comprend :
(1) un premier composant (A) constitué par une composition contenant en milieu aqueux au moins un réducteur thiolé;
(2) un second composant (B) constitué par une composition contenant en milieu aqueux au moins un polymère épaississant en dispersion aqueuse ou huileuse ou en émulsion inverse;

les composants (A) et (B) étant destinés à être mélangés l'un avec l'autre au moment de l'emploi en vue d'obtenir une composition réductrice prête à l'emploi destinée à êre appliquée sur des cheveux en vue de réduire les liaisons disulfures de ceux-ci.

Préférentiellement, le réducteur est choisi parmi l'acide thioglycolique, l'acide thiolactique, la cystéine, la cystéamine, le thioglycérol et le thioglycolate de glycérol ou l'un de leurs sels cosmétiquement acceptables tels que plus particulièrement les chlorhydrates, les bromhydrates, les citrates, les acétates et les sulfates.

Le réducteur est utilisé dans des proportions suffisantes pour réduire les liaisons disulfures et, de préférence, dans des proportions comprises entre 1 et 25% en particulier de 3 à 25% en poids par rapport au poids total de la composition prête à l'emploi.

Les polymères épaississants du composant (B) sont choisis de préférence parmi
- les copolymères d'acrylate d'ammonium/acrylamide, en émulsion inverse E/H tel que le BOZEPOL C commercialisé par HOECHST;
- les copolymères acrylamide/acrylamido-2-méthyl propane sulfonique, en émulsion inverse tel que le SEPIGEL 305 commercialisé par SEPPIC;
- les copolymères acrylate de sodium/acrylamide, en émulsion inverse tel que le SEPIGEL 901 commercialisé par SEPPIC;
- les copolymères chlorure de méthacrylate de triméthyl éthyl ammonium/acrylate en dispersion dans l'huile tel que le SALCARE SC 92 commercialisé par ALLIED COLLOIDS;
- les homopolymères de chlorure de méthacrylate d'éthyle triméthyl ammonium en dispersion dans l'huile tel que le SALCARE SC 95 commercialisé par ALLIED COLLOIDS;
- les hydroxypropyl méthylcellulose en dispersion aqueuse tel que l'AQU D-3295A commercialisés par HERCULES.

Le polymère épaississant est présent dans la composition (B) dans des proportions telles que la composition prête à l'emploi résultant du mélange des composants (A) et (B) présente une viscosité suffisante pour ne pas couler sur le cuir chevelu et/ou pour maintenir les déformations imprimées aux cheveux.

Les polymères épaississants sont utilisés de préférence dans des proportions comprises entre 0,1 et 30% en poids par rapport au poids total de la composition prête à l'emploi.

Le composant (A) et/ou le composant (B) susmentionnés peuvent contenir en outre un ou plusieurs agents alcalins.

Les agents alcalins peuvent notamment être choisis parmi la monoéthanolamine, la diéthanolamine, la triéthanolamine, l'isopropanolamine, la propanediamine-1,3, un carbonate de bicarbonate alcalin ou d'ammonium, un carbonate organique tel que le carbonate de guanidine ou encore un hydroxyde alcalin, utilisés seul ou en mélange.

Le pH du composant (A) et celui du composant (B) peuvent être ajustés de façon à obtenir un pH de la composition prête à l'emploi compris entre 7,0 et 11,0.

L'agent réducteur peut également contenir, soit dans son composant (A), soit dans son composant (B) soit dans le mélange prêt à l'emploi, des agents tensio-actifs, des agents traitants de nature anionique, non-ionique ou amphotère.

Les agents tensio-actifs utilisés sont ceux couramment utilisés dans les compositions réductrices de permanentes et peuvent être de type non-ionique, anionique, cationique ou amphotère. On peut notamment citer les alkylsulfates, les alkylbenzènesulfates, les alkyléthersulfates, les alkylsulfonates, les sels d'ammonium quaternaire, les alkylbétaïnes, les alkylphénols oxyéthylénés, les alcanolamides d'acides gras, les esters d'acides gras oxyéthylénés ainsi que des tensio-actifs non-ioniques de la famille des hydroxypropyléthers.

Ces agents tensio-actifs sont généralement utilisés dans des proportions telles que dans la composition résultant du mélange des composants (A) et (B), leur proportion maximale soit de l'ordre de 30% en poids, et de préférence comprise entre 0,5 et 10% en poids par rapport au poids total de la composition.

On peut utiliser comme agents traitants des silicones volatiles ou non, linéaires ou cycliques ou leurs mélanges, les polydiméthylsiloxanes, les polyorganosiloxanes quaternisés, ceux décrits dans la demande de brevet français 2 535 730, les polyorganosiloxanes à groupement amino-alkyle modifiés par des groupements alcoxycarbonylakyle tels que décrits dans le brevet US-A-4 749 732, les polyorganosiloxanes tels que les copolymères de polydiméthylsiloxane-polyoxyalkyle tel que le diméthicone copolyol, les polydiméthylsiloxanes à groupements terminaux stéaroxy-(stéaroxydiméthicone), les copolymères polydiméthylsiloxane dialkylammonium acétate ou copolymères polydiméthylsiloxane polyalkylétaîne décrit dans GB-A-2 197 352, des polysiloxanes organomodifiés par des groupements mercapto ou mercapto-alkyle tels que décrits dans FR-B-1 530 369 et EP-A-0 295 780, ainsi que des silanes tels que le stéroxy-triméthylsilane.

On peut également utiliser d'autres ingrédients traitants tels que des cires, des polymères choisis parmi les polymères cosmétiquement acceptables qui peuvent être des polymères cationiques, anioniques, non-ioniques ou amphotères, des agents de gonflement et de pénétration permettant de renforcer l'efficacité du réducteur tels que le diméthylisosorbitol, l'urée et ses dérivés, la pyrrolidone, les n-alkylpyrrolidone, la thiamorpholinone, les alkyléthers d'alkylèneglycol ou de dialkylèneglycol tels que par exemple le monométhyléthyléther de propylèneglycol, le monométhyléther de dipropylèneglycol, des alcanediols en C₃-C₆ tels que par exemple le propanediol-1,2, l'imidazolidinone-2, ainsi que d'autres composés tels que des alcools gras, des dérivés de lanoline, des céramides et notamment des céramides elles-mêmes, les glycocéramides, les pseudocéramides décrits notamment dans FR-A-95/1399, et dans DOWNING Journal of Lipid Research, Vol. 35, p. 2060, 1994, ou dans FR-A-2 673 197, EP-A-0227994, WO-94/07844, WO-92/05674, des ingrédients actifs tels que l'acide pantoténique ou le panthénol, des agents anti-chute, des agents anti-pelliculaires, des agents de mise en suspension, des agents séquestrants, des agents opacifiants, des colorants, des filtres solaires, ainsi que des parfums et des conservateurs.

Avant l'emploi, le composant (B) défini ci-dessus est mélangé avec le composant (A) défini ci-dessus.

Préférentiellement, on mélange 1 à 99% en poids en particulier 60 à 98,5% en poids par rapport au poids total de composant (A) contenant un réducteur avec 99 à 1% en particulier 1,5 à 40% en poids de composant (B) contenant un polymère épaississant en dispersion aqueuse ou huileuse ou en émulsion inverse.

Le procédé conforme à l'invention est essentiellement caractérisé par le fait
- qu'on procède au préalable à un mélange d'une composition contenant en milieu aqueux un réducteur telle que définie ci-dessus avec une composition contenant un polymère épaississant en dispersion aqueuse ou huileuse ou en émulsion inverse telle que définie ci-dessus;
- qu'on applique la composition ainsi obtenue sur la chevelure mouillée ou non avant, pendant ou après l'étape de mise sous tension des cheveux par un moyen mécanique ou mis en forme par tous moyens manuels;
- qu'on applique après un temps de pose suffisant pour permettre la réduction des liaisons disulfures du cheveux, et après un rinçage éventuel une composition de fixation contenant au moins un agent oxydant;
- qu'on procède après un temps de pose suffisant au rinçage final.

L'étape de mise sous tension des cheveux peut être mise en oeuvre par tous moyens mécaniques appropriés et connus tels que par exemple des rouleaux, bigoudis, etc...

Grâce aux compositions de la présente invention, il est possible de mettre en oeuvre le procédé sans utiliser de matériel de mise sous tension des cheveux, c'est-à-dire en appliquant simplement la composition à l'aide des doigts ou du peigne ce qui permet de sculpter la chevelure afin de maintenir les cheveux dans une position désirée telle que des boucles, des crans ou des épis.

Selon une étape facultative du procédé de l'invention, on peut, après application de la composition réductrice, soumettre la chevelure à un traitement thermique par chauffage à température comprise entre 30 et 60° C. Ce chauffage permet éventuellement d'ajuster le degré final de frisure du cheveux.

Dans la pratique, cette opération peut être conduite au moyen d'un casque de coiffure, d'un sèche cheveux, d'un dispensateur de rayons infrarouges et d'autres appareils chauffants classiques.

Il est également possible, bien entendu, de travailler à température ambiante.

Selon une forme particulière de réalisation de l'invention, il est possible de ne pas procéder à l'étape de rinçage après réduction notammnent lorsque les cheveux ont été mis en forme par des moyens autres que des moyens mécaniques.

De façon générale, avant de procéder au rinçage ou à l'application de la composition oxydante, on laisse reposer pendant quelques minutes, généralement entre 2 et 30 minutes, et de préférence entre 5 et 20 minutes, la chevelure sur laquelle a été appliquée la composition réductrice, ceci de façon à bien laisser le temps au réducteur d'agir correctement sur les cheveux.

Pendant cette phase d'attente, on prend soin que les cheveux ne sèchent pas complètement et restent ainsi humides jusqu'au moment de la mise en oeuvre de l'étape suivante. On peut ainsi utiliser dans ce but des bonnets ou des gels de protection.

Les agents oxydants utilisables dans les compositions de fixation peuvent notamment être choisis parmi le peroxyde d'hydrogène ou eau oxygénée, le peroxyde d'urée, les bromates de métaux alcalins; les persels tels que les perborates et persulfates, et les enzymes telles que les peroxydases et les oxydo-réductases à deux électrons. L'utilisation du peroxyde d'hydrogène ou des enzymes est particulièrement préférée.

La concentration en eau oxygénée peut varier de 1 à 10 volumes, mais de préférence est de l'ordre de 8 volumes.

La concentration en bromates alcalins est de 1 à 12% et celle en persels de 0,1 à 15% en poids par rapport au poids total de la composition oxydante.

Le pH de la composition oxydante peut varier entre 2 et 7, et de préférence est compris entre 3 et 6 notamment dans le cas de l'eau oxygénée.

L'eau oxygénée peut être stabilisée, par exemple par la phénacétine, l'acétaniline, les phosphates mono- et trisodiques ou par les sulfates d'hydroxy-8 chinoléïne.

Les compositions fixatrices ou oxydantes peuvent en outre également contenir des agents alcalins, des agents tensioactifs ou des agents traitants tels que définis ci-dessus.

Dans le cas où une mise sous tension par un moyen mécanique a été effectuée, on peut retirer de la chevelure les moyens mécaniques ou les bigoudis et analogues qui maintenaient les cheveux sous tension selon la forme désirée tout au long du traitement, avant ou après l'étape de fixation.

Le temps de pose de la composition de fixation peut varier de 5 à 30 mn en particulier de 5 à 15 mn.

Les exemples suivants sont destinés à illustrer l'invention sans présenter un caractère limitatif:

### EXEMPLE 1

### 1) Partie A

| | |
|---|---|
| SEPIGEL 305 (SEPPIC) (copolymère acrylate de sodium/acrylamide en émulsion inverse dans isoparaffine/eau) | 15 g |

### 2) Partie B

- Acide thioglycolique 9 g
- Acide diéthylène triamine pentacétique, 0,4 g
   sel pentasodique, en solution aqueuse à 40%
- ammoniaque qs PH 8,4
- Parfum 0,5 g
- Alcool oléique oxyéthyléné(20 OE) 1 g
- Eau déminéralisée qs 85 g

Au moment de l'emploi, on introduit dans un shaker la partie A et la partie B. On secoue et on obtient immédiatement un gel crème blanc homogène, prêt pour l'application.

### EXEMPLE 2

### 1) Partie A

| | |
|---|---|
| BOZEPOL C (HOECHST) (copolymère acrylate d'ammonium / acrylamide (95/5) en émulsion inverse E/H) | 6 g |

### 2) Partie B

- Acide thiolactique 5 g
- Acide diéthylènetriamine pentacétique, 0,2 g
   sel pentasodique, en solution aqeuse à 40%
- Ammoniaque qs pH 7,9
- Carbonate d'ammonium 5 g
- Parfum 0,5 g
- Alcool oléique oxyéthyléné(20 OE) 1 g
- Eau déminéralisée sqp 85 g

Au moment de l'emploi, on introduit dans un shaker la partie A et la partie B. On secoue et on obtient immédiatement un gel crème blanc homogène, prêt pour l'application.

## Revendications

1. Agent réducteur destiné à être utilisé dans un procédé de déformation de permanente des cheveux, **caractérisé par le fait qu'**il comprend au moins :
- un premier composant constitué par une composition contenant en milieu aqueux au moins un réducteur thiolé,
- un second composant constitué par une composition contenant en milieu aqueux au moins un polymère épaississant en dispersion aqueuse ou huileuse ou en émulsion inverse choisi parmi les copolymères d'acrylate d'ammonium/acrylamide, les copolymères acrylamide/acrylamido 2-méthyl propane sulfonique, les copolymères d'acrylate de sodium/acrylamide, les copolymères de chlorure de méthacrylate de triméthyl éthyl ammonium/acrylate, les homopolymères de chlorure de méthacrylate d'éthyl triméthyl ammonium réticulés et les hydroxypropyl méthylcellulose;
le premier et le second composant étant destinés à être mélangés l'un avec l'autre au moment de l'emploi en vue d'obtenir une composition réductrice prête à l'emploi destinée à être appliquée sur des cheveux en vue de réduire les liaisons disulfures de ceux-ci.

2. Agent selon la revendication 1, **caractérisé par le fait que** le réducteur thiolé du premier composant est choisi parmi l'acide thioglycolique, l'acide thiolactique, la cystéine, la cystéamine et le thioglycolate de glycérol.

3. Agent selon la revendication 1 ou 2 **caractérisé par le fait que** le réducteur thiolé est présent dans des proportions telles que la concentration en réducteurs dans la composition prête à l'emploi soit comprise entre 1 et 25% en poids par rapport au poids total de la composition prête à l'emploi.

4. Agent selon l'une quelconque des revendications 1 à 3, **caractérisé par le fait que** le polymère épaississant est présent dans des proportions après mélange comprises entre 0,1 et 30% en poids par rapport au poids total de la composition prête à l'emploi.

5. Agent selon l'une quelconque des revendications 1 à 4, **caractérisé par le fait que** la composition contenant en milieu aqueux au moins un réducteur thiolé et/ou la composition contenant en milieu aqueux au moins un polymère épaississant en dispersion ou en émulsion inverse et/ou la composition prête à l'emploi contient en outre un ou plusieurs agents alcalins.

6. Agent selon l'une quelconque des revendications 1 à 5, **caractérisé par le fait que** la composition contenant en milieu aqueux au moins un réducteur thiolé et/ou la composition contenant en milieu aqueux au moins un polymère épaississant en dispersion ou en émulsion inverse et/ou la composition prête à l'emploi contient en outre, des agents tensio-actifs anioniques, non-ioniques, cationiques ou amphotères.

7. Agent selon l'une quelconque des revendications 1 à 6, **caractérisé par le fait que** la composition contenant en milieu aqueux au moins un réducteur thiolé et/ou la composition contenant en milieu aqueux au moins un polymère épaississant en dispersion ou en émulsion inverse et/ou la composition prête à l'emploi contient en outre, des agents traitants choisis parmi les silicones, des cires, des polymères, des agents de gonflement et de pénétration, des alcools gras, des dérivés de lanoline, des céramides, des ingrédients actifs, des agents anti-chute, anti-pelliculaires, des agents de mise en suspension, des agents séquestrants, des agents opacifiants, des colorants, des filtres solaires, des conservateurs, des parfums.

8. Procédé de déformation permanente des cheveux, **caractérisé par le fait**
- **qu'**on procède au préalable à un mélange d'une composition contenant en milieu aqueux un réducteur telle que définie dans l'une quelconque des revendications 1 à 7 avec une composition contenant un polymère épaississant en dispersion huileuse ou aqueuse ou en émulsion inverse telle que définie dans l'une quelconque des revendications 1 à 7;
- **qu'**on applique la composition prête à l'emploi ainsi obtenue sur la chevelure avant, pendant ou après l'étape de mise sous tension des cheveux par un moyen mécanique ou mis en forme par tous moyens manuels;
- **qu'**on applique après un temps de pose suffisant et un éventuel rinçage pour permettre la réduction des liaisons disulfures du cheveux, une composition de fixation contenant au moins un agent oxydant;
- **qu'**on procède après un temps de pose suffisant pour permettre la déformation permanente, au rinçage.

9. Procédé selon la revendication 8, **caractérisé par le fait que** l'on mélange 1% à 99% en poids par rapport au poids total de la composition contenant en milieu aqueux un réducteur avec 99 à 1% en poids par rapport au poids total de la composition prête à l'emploi de composition contenant un polymère épaississant en dispersion ou en émulsion inverse.

10. Procédé selon la revendication 8 ou 9, **caractérisé par le fait que** le pH de la composition prête à l'emploi est un pH compris entre 7,0 et 11,0.

11. Procédé selon l'une quelconque des revendications 8 à 10, **caractérisé par le fait que** la composition réductrice est maintenue au contact des cheveux pendant 2 à 30 mn.

12. Procédé selon la revendication 11, **caractérisé par le fait que** les cheveux sont rincés avant l'application de la composition oxydante.

13. Procédé selon l'une quelconque des revendications 8 à 12, **caractérisé par le fait que** la composition oxydante contient un agent oxydant choisi parmi le peroxyde d'hydrogène, le peroxyde d'urée, les bromates de métaux alcalins; les persels tels que les perborates et persulfates et les enzymes telles que les peroxydases et les oxydo-réductases à deux électrons.

14. Procédé selon l'une quelconque des revendications 8 à 13, **caractérisé par le fait que** l'on maintient la composition oxydante au contact des cheveux et que l'on rince ensuite à l'eau.

## Claims

1. Reducing agent intended to be used in a process for permanently reshaping the hair, **characterized in that** it comprises at least :
- a first component consisting of a composition containing at least one thiol-bearing reducing agent in aqueous medium,
- a second component consisting of a composition containing at least one thickening polymer in aqueous medium, as an aqueous or oily dispersion or as a reverse emulsion chosen from ammonium acrylate/ acrylamide copolymers, acrylamide/2-acrylamidomethylpropanesulphonic copolymers, the sodium acrylate/acrylamide copolymers, copolymers of trimethylethylammonium methacrylate chloride/acrylate, homopolymers of crosslinked ethyltrimethylammonium methacrylate chloride and hydroxypropylmethylcelluloses,
the first and second component being intended to be mixed with each other at the time of use in order to obtain a ready-to-use reducing composition intended to be applied to the hair in order to reduce the hair's disulphide linkages.

2. Agent according to Claim 1, **characterized in that** the thiol-bearing reducing agent in the first component is chosen from thioglycolic acid, thiolactic acid, cysteine, cysteamine and glyceryl thioglycolate.

3. Agent according to Claim 1 or 2, **characterized in that** the thiol-bearing reducing agent is present in proportions such that the concentration of reducing agents in the ready-to-use composition is between 1 and 25% by weight relative to the total weight of the ready-to-use composition.

4. Agent according to any one of Claims 1 to 3, **characterized in that** the thickening polymer is present in proportions, after mixing, of between 0.1 and 30% by weight relative to the total weight of the ready-to-use composition.

5. Agent according to any one of Claims 1 to 4, **characterized in that** the composition containing at least one thiol-bearing reducing agent in aqueous medium and/or the composition containing at least one thickening polymer in aqueous medium, as a dispersion or as a reverse emulsion, and/or the ready-to-use composition also contains one or more alkaline agents.

6. Agent according to anyone of Claims 1 to 5, **characterized in that** the composition containing at least one thiol-bearing reducing agent in aqueous medium and/or the composition containing at least one thickening polymer in aqueous medium, as a dispersion or as a reverse emulsion, and/or the ready-to-use composition also contains anionic, nonionic, cationic or amphoteric surfactants.

7. Agent according to anyone of Claims 1 to 6, **characterized in that** the composition containing at least one thiol-bearing reducing agent in aqueous medium and/or the composition containing at least one thickening polymer in aqueous medium, as a dispersion or as a reverse emulsion, and/or the ready-to-use composition also contains treating agents chosen from silicones, waxes, polymers, swelling agents, penetrating agents, fatty alcohols, lanolin derivatives, ceramides, active ingredients, agents for preventing hair loss, antidandruff agents, suspending agents, sequestering agents, opacifiers, dyes, sunscreens, preserving agents and fragrances.

8. Process for permanently reshaping the hair, **characterized in that**:
- a composition containing a reducing agent as defined in anyone of Claims 1 to 7 in aqueous medium is premixed with a composition containing a thickening polymer as an aqueous or oily dispersion or as a reverse emulsion as defined in any one of Claims 1 to 7,
- the ready-to-use composition thus obtained is applied to hair before, during or after the step of placing the hair under tension by a mechanical means or shaping by any manual means;
- after leaving the composition on the hair for a period of time which is sufficient to allow the reduction of the disulphide linkages of the hair, and after an optional rinsing, a fixing composition containing at least one oxidizing agent is applied;
- after leaving the fixing composition on the hair for a period of time which is sufficient to allow permanent reshaping, rinsing is carried out.

9. Process according to Claim 8, **characterized in that** 1% to 99% by weight, relative to the total weight of the composition containing a reducing agent in aqueous medium, are mixed with 99 to 1% by weight, relative to the total weight of the ready-to-use composition, of composition containing a thickening polymer, as a dispersion or as a reverse emulsion.

10. Process according to Claim 8 or 9, **characterized in that** the pH of the ready-to-use composition is a pH of between 7.0 and 11,0.

11. Process according to anyone of Claims 8 to 10, **characterized in that** the reducing composition is kept in contact with the hair for 2 to 30 min.

12. Process according to Claim 11, **characterized in that** the hair is rinsed before applying the oxidizing composition.

13. Process according to anyone of Claims 8 to 12, **characterized in that** the oxidizing composition contains an oxidizing agent chosen from hydrogen peroxide, urea peroxide, alkali metal bromates, persalts such as perborates and persulphates, and enzymes such as peroxidases and two-electron oxidoreductases.

14. Process according to anyone of Claims 8 to 13, **characterized in that** the oxidizing composition is kept in contact with the hair and rinsing is then carried out with water.

## Patentansprüche

1. Reduktionsmittel, das für die Verwendung in einem Verfahren zur permanenten Verformung der Haare vorgesehen ist, **dadurch gekennzeichnet, dass** es mindestens umfasst:
• eine erste Komponente, die aus einer Zusammensetzung besteht, die in wässrigem Medium mindestens eine reduzierende Thiolverbindung enthält,
• eine zweite Komponente, die aus einer Zusammensetzung besteht, die in wässrigem Medium mindestens ein verdickendes Polymer in wässriger oder öliger Dispersion oder in inverser Emulsion enthält, das unter den Ammoniumacrylat/Acrylamid-Copolymeren, den Acrylamid/Acrylamido-2-methylpropansulfonsäure-Copolymeren, den Natriumacrylat/Acrylamid-Copolymeren, den Trimethylammonioethylmethacrylatchlorid/Acrylat-Copolymeren, den vernetzten Homopolymeren aus Trimethylammonioethylmethacrylatchlorid und den Hydroxypropylmethylcellulosen ausgewählt ist,
wobei die erste und die zweite Komponenten dafür vorgesehen sind, zum Zeitpunkt der Anwendung miteinander vermischt zu werden, um eine gebrauchsfertige reduzierende Zusammensetzung zu erhalten, die dafür vorgesehen ist, auf die Haare aufgetragen zu werden, um die Disulfid-Bindungen der Haare zu reduzieren.

2. Mittel nach Anspruch 1, **dadurch gekennzeichnet, dass** die reduzierende Thiolverbindung der ersten Komponente unter Thioglykolsäure, Thiomilchsäure, Cystein, Cysteamin und Thioglycolsäureglycerinester ausgewählt ist.

3. Mittel nach einem der Ansprüche 1 oder 2, **dadurch gekennzeichnet, dass** die reduzierende Thiolverbindung in einem solchen Mengenanteil enthalten ist, dass die Konzentration der gebrauchsfertigen Zusammensetzung an reduzierenden Verbindungen 1 bis 25 Gew.-%, bezogen auf das Gesamtgewicht der gebrauchsfertigen Zusammensetzung, beträgt.

4. Mittel nach einem der Ansprüche 1 bis 3, **dadurch gekennzeichnet, dass** das verdickende Polymer nach dem Mischen in einem Mengenanteil enthalten ist, der im Bereich von 0,1 bis 30 Gew.-%, bezogen auf das Gesamtgewicht der gebrauchsfertigen Zusammensetzung, liegt.

5. Mittel nach einem der Ansprüche 1 bis 4, **dadurch gekennzeichnet, dass** die Zusammensetzung, die in wässrigem Medium mindestens eine reduzierende Thiolverbindung enthält, und/oder die Zusammensetzung, die in wässrigem Medium mindestens ein verdickendes Polymer in Dispersion oder in inverser Emulsion enthält, und/oder die gebrauchsfertige Zusammensetzung außerdem ein oder mehrere alkalische Mittel enthält.

6. Mittel nach einem der Anspräche 1 bis 5, **dadurch gekennzeichnet, dass** die Zusammensetzung, die in wässrigem Medium mindestens eine reduzierende Thiolverbindung enthält, und/oder die Zusammensetzung, die in wässrigem Medium mindestens ein verdickendes Polymer in Dispersion oder in inverser Emulsion enthält, und/oder die gebrauchsfertige Zusammensetzung außerdem anionische, nicht-ionische, kationische oder amphotere grenzflächenaktive Stoffe enthält.

7. Mittel nach einem der Anspräche 1 bis 6, **dadurch gekennzeichnet, dass** die Zusammensetzung, die in wässrigem Medium mindestens eine reduzierende Thiolverbindung enthält, und/oder die Zusammensetzung, die in wässrigem Medium mindestens ein verdickendes Polymer in Dispersion oder in inverser Emulsion enthält, und/oder die gebrauchsfertige Zusammensetzung außerdem Behandlungsmittel enthält, die unter Siliconen, Wachsen, Polymeren, Quellmitteln und Penetrationshilfsmitteln, Fettalkoholen, Lanolinderivaten, Ceramiden, Wirkstoffen, Wirkstoffen gegen Haarausfall, Antischuppenmitteln, Suspendierhilfen, Maskierungsmitteln, Trübungsmitteln, Farbmitteln, Sonnenschutzfiltern, Konservierungsmitteln, Parfüms ausgewählt sind.

8. Verfahren zur permanenten Verformung der Haare, **dadurch gekennzeichnet, dass**
• zunächst eine Zusammensetzung, die in wässrigem Medium eine reduzierende Verbindung enthält, die wie in einem der Ansprüche 1 bis 7 definiert ist, mit einer Zusammensetzung, die ein verdickendes Polymer in wässriger oder öliger Dispersion oder in inverser Emulsion enthält, die wie in einem der Ansprüche 1 bis 7 definiert ist, vermischt wird;
• die so erhaltene gebrauchsfertige Zusammensetzung vor, während oder nach dem Schritt, in dem die Haare mit einem mechanischen Mittel unter Spannung gesetzt werden oder mit einem beliebigen manuellen Mittel in eine Form gebracht werden, auf das Haar aufgetragen wird;
• nach einer Einwirkungszeit und einem gegebenenfalls durchgeführten Spülen der Haare, die ausreichend ist, um die Reduktion der Disulfid-Bindungen der Haare zu ermöglichen, eine fixierende Zusammensetzung aufgetragen wird, die mindestens ein Oxidationsmittel enthält;
• nach einer Einwirkungszeit, die ausreichend ist, um die permanente Verformung zu ermöglichen, die Haare gespült werden.

9. Verfahren nach Anspruch 8, **dadurch gekennzeichnet, dass** 1 bis 99 Gew.-%, bezogen auf das Gesamtgewicht, der Zusammensetzung, die in wässrigem Medium eine reduzierende Verbindung enthält, mit 99 bis 1 Gew.-%, bezogen auf das Gesamtgewicht der gebrauchsfertigen Zusammensetzung, der Zusammensetzung, die ein verdickendes Polymer in Dispersion oder in inverser Emulsion enthält, vermischt werden.

10. Verfahren nach Anspruch 8 oder 9, **dadurch gekennzeichnet, dass** der pH-Wert der gebrauchsfertigen Zusammensetzung ein pH-Wert ist, der im Bereich von 7,0 bis 11,0 liegt.

11. Verfahren nach einem der Ansprüche 8 bis 10, **dadurch gekennzeichnet, dass** die reduzierende Zusammensetzung während eines Zeitraums von 2 bis 30 min mit den Haaren in Kontakt gehalten wird.

12. Verfahren nach Anspruch 11, **dadurch gekennzeichnet, dass** die Haare gespült werden, bevor die oxidierende Zusammensetzung aufgetragen wird.

13. Verfahren nach einem der Ansprüche 8 bis 12, **dadurch gekennzeichnet, dass** die oxidierende Zusammensetzung ein Oxidationsmittel enthält, das unter Wasserstoffperoxid, Harnstoffperoxid, Alkalimetallbromaten, Persalzen, wie den Perboraten und den Persulfaten, und Enzymen, wie den Peroxidasen und den Oxidoreduktasen, die zwei Elektronen übertragen, ausgewählt ist.

14. Verfahren nach einem der Ansprüche 8 bis 13, **dadurch gekennzeichnet, dass** die oxidierende Zusammensetzung im Kontakt mit den Haaren gehalten wird und dass die Haare anschließend gespült werden.
